# EUROPEAN PATENT SPECIFICATION

(11) **EP 1 453 949 B1**
(45) Date of publication and mention of the grant of the patent: **09.08.2006**
(21) Application number: 02786667.2
(22) Date of filing: 06.11.2002
(51) Int. Cl.: C12N 5/00, C12N 5/02, C12N 5/06

(54) **PRODUCTION OF CELL SUSPENSIONS**
HERSTELLUNG VON ZELLSUSPENSIONEN
PRODUCTION DE SUSPENSIONS CELLULAIRES

(30) Priority: 09.11.2001 US 345522 P
(43) Date of publication of application: 08.09.2004
(73) Proprietor: Viacell, Inc., Worcester, MA 01605 (US)
(72) Inventor: KRAUS, Morey, Jefferson, MA 01522 (US); PANG, Lizhen, Tucson, AZ 85718 (US)
(74) Representative: Gervasi, Gemma
(86) International application number: PCT/US2002/035553
(87) International publication number: WO 2003/042113

(56) References cited:
- WO-A-99/07831
- US-A- 5 525 229
- US-A- 5 707 417
- US-A- 5 888 307
- KRAUS M ET AL: "Negative impact of CD14 cells on the expansion of Umbilical Cord Blood Hematopoeitic Progenitor Cells in serum free cultures" BLOOD, vol. 98, no. 11 Part 2, 16 November 2001 (2001-11-16), page 337b, XP009038891 & 43RD ANNUAL MEETING OF THE AMERICAN SOCIETY OF HEMATOLOGY, PART 2; ORLANDO, FLORIDA, USA; DECEMBER 07-11, 2001 ISSN: 0006-4971
- KRAUS M ET AL: "Selective expansion of distinct populations in Umbilical Cord Blood using various antibody cocktail selection strategies" BLOOD, vol. 98, no. 11 Part 2, 16 November 2001 (2001-11-16), page 338b, XP009038893 & 43RD ANNUAL MEETING OF THE AMERICAN SOCIETY OF HEMATOLOGY, PART 2; ORLANDO, FLORIDA, USA; DECEMBER 07-11, 2001 ISSN: 0006-4971
- MAPARA M Y ET AL: "Combined positive/negative selection in stem cell transplantation: Perspectives in graft engineering" INFUSIONSTHERAPIE UND TRANSFUSIONMEDIZIN, vol. 26, no. 3, May 1999 (1999-05), pages 140-145, XP009038908 ISSN: 1019-8466
- WILDER P T ET AL: "Selective expansion of cord blood LIN-hematopoietic stem cells" BLOOD, vol. 96, no. 11 Part 1, 16 November 2000 (2000-11-16), page 776a, XP009038895 & 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- KAUFMAN C L ET AL: "PHENOTYPIC CHARACTERIZATION OF A NOVEL BONE MARROW-DERIVED CELL THAT FACILITATES ENGRAFTMENT OF ALLOGENEIC BONE MARROW STEM CELLS" BLOOD, W.B. SAUNDERS, PHILADELPHIA, VA, US, vol. 84, no. 8, 1994, pages 2436-2446, XP000909509 ISSN: 0006-4971

## Description

### BACKGROUND OF THE INVENTION

This invention relates to the production, by expansion and selection methods, of cell populations that can be infused into patients, for example, to engraft patients, e.g., cancer patients who have had treatment that resulted in the depletion of their bone marrow. Other uses of such cell populations include supplying cells as supplements for genetic defects, as elements for regeneration of tissue, as carriers of therapeutic genes, and as agents used for immune enhancement.

In the case of engraftment, cancer patients can have their bone marrow reconstituted by the administration of bone marrow obtained from donors, or by the administration of suspensions of relatively immature, and therefore, pluripotent, cells, including stem cells, obtained from other sources, such as umbilical cord blood. Methods have been devised to expand such cells in culture, while selecting the desired, or "target" cells, such as stem cells. A method for achieving this is described in Kraus, U.S. Patent No. 5,925,567, hereby incorporated by reference. Such systems typically employ cytokines and other growth factors to ensure that the resulting cells suspension is enriched in the target cell type. In addition, such methods have employed supporting cells such as stromal cells, which can enhance the expansion of the target cells.

### SUMMARY OF THE INVENTION

The invention features a method for producing an engraftable cell suspension, wherein the method includes the steps of:
a) culturing a first population of cells that contains multiple cell types, including a first sub-population of cells of a type suitable for engraftment, under conditions that cause that expansion of the first sub-population of cells, wherein the population of cells further comprises a second sub-population of cells that either inhibit the expansion, or grow less well under these conditions than the cells of the first sub-population, and that are capable of enhancing engraftment;
b) before, during, or following the culturing of step (a), removing cells of the second sub-population;
c) preserving the removed cells of the second sub-population; and
d) following expansion of cells of the first sub-population, combining cells of the first sub-population with cells of the preserved second sub-population to form the engraftable cell suspension.

In certain embodiments, step (d) is performed after expansion of the first sub-population of cells has been completed. The method can further include the removal of dead cells, and can also include the step of introducing a third sub-population of engraftment-enhancing cells or expansion-enhancing cells to the suspension, at any stage in the process. In cases where the cells of the third sub-population enhance expansion of the first sub-population, the cells of the third sub-population are added to the culture containing the first sub-population of cells prior to the completion of the expansion, in order to enhance that process. The cells of the second sub-population that are removed initially can be expanded prior to being recombined with the cells of the first sub-population. Removal of the cells of the second sub-population can be effected by use of selection elements, e.g. antibodies, cell adhesion molecules (CAMs), and/or ligands to various growth factors. Positive or negative selection can be used, e.g. as described in U.S. Patent No. 5,925,567. Repeated or continuous selection of one or more sub-populations can be performed, with the criteria for selection being varied over time if desired.

The invention takes advantage of the discovery that some sub-populations of cells, e.g., cells that express the surface antigen CD14, while undesirably inhibiting the expansion of desired cells, e.g. CD45+ cells such that their removal enhances expansion, may enhance characteristics of the engraftable cell suspension if added back following expansion. A further important insight is that sub-populations that are removed, temporarily, can be expanded prior to being added back to the cell suspension, under conditions which may be different from those in the original culture, and more conducive to expansion of the removed sub-population. Because a starting cell culture may contain a number of different cell types, any of which can be removed at any stage of the process, expanded, and added back in any proportion, the characteristics of the final cell suspension can be adjusted infinitely using the methods of the invention.

Certain cell sub-populations enhance the growth of the primary culture and/or the sub-population, and/or enhance the properties (potency, efficacy, etc.) of the cell suspension. Growth enhancement and/or enhancement of the properties of the final cell suspension can be obtained by recombining the primary culture, sub-populations and/or progeny of sub-populations at various times during or after expansion.

Other features and advantages of the invention will be apparent from the detailed description and drawings, and from the claims.

### DESCRIPTION OF DRAWINGS

Fig. 1 is a graph showing the effect of various antibody cocktails on the expansion potential of a cell culture.
Fig. 1A is a graph illustrating the effect of various concentrations ofCD14+ cells on the expansion potential of a cell culture.
Fig. 2 is a highly enlarged diagrammatic view of a system for positive selection of a target cell. Fig. 2A is a highly enlarged diagrammatic view of a system for negative selection of a non-target cell.
Figs. 3a-3d are schematic diagrams of various modes of operation of systems according to different embodiments of the invention.

### DETAILED DESCRIPTION

The invention features methods of forming cell products having defined profiles by selecting a sub-population from a primary culture, or from an initial cell suspension not in culture, and using the sub-population, a component of the sub-population, and/or the primary culture to make one or more cell products. In some cases, the sub-population may ultimately be added to the primary culture and/or the cell suspension to form the final product. Examples of initial cell suspensions include bone marrow, cord blood, peripheral blood, and suspensions of cells obtained from human mesodermal, ectodermal, and endodermal tissues, such as pancreatic, hepatic, neural, nephrotic, dermal, muscle and cardiac tissue. Examples of primary cultures include cultures of cells of a type suitable for engraftment, such as hematopoietic stem cells.

The methods of the invention may include forming cell products by combining cells of the sub-population with other cells. For example, the sub-population may be returned to the primary culture after further expansion of the primary culture. Alternatively, the sub-population may be cultured separately and eventually returned to the primary culture, or its progeny may be returned to the primary culture (either with or without intervening further expansion of the primary culture). A second sub-population may be removed from the first sub-population, and then the first or second sub-population may be returned to the primary culture, either before or after further expansion of the primary culture and/or the sub-population that is being returned. The sub-population can also be added to a cell suspension from which the primary culture or the sub-population was obtained, e.g., one of the initial cell suspensions discussed above.

In other implementations, methods include, during expansion of a sub-population, adding cells of the same type as those in the sub-population to the sub-population to fuel expansion of the sub-population, and/or removing a sub-population and combining it with other cells not of the primary culture.

In some implementations, in addition to the selection steps discussed above, an undesirable sub-population is removed from the primary culture and discarded.

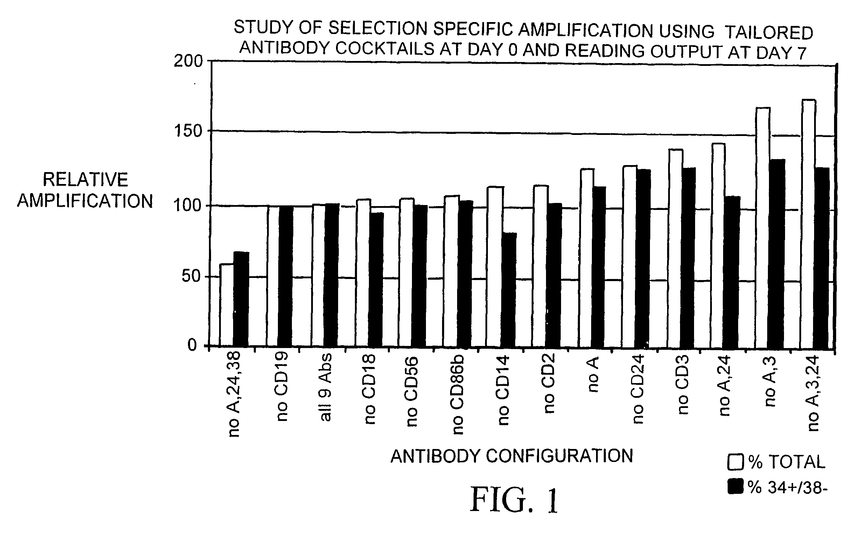

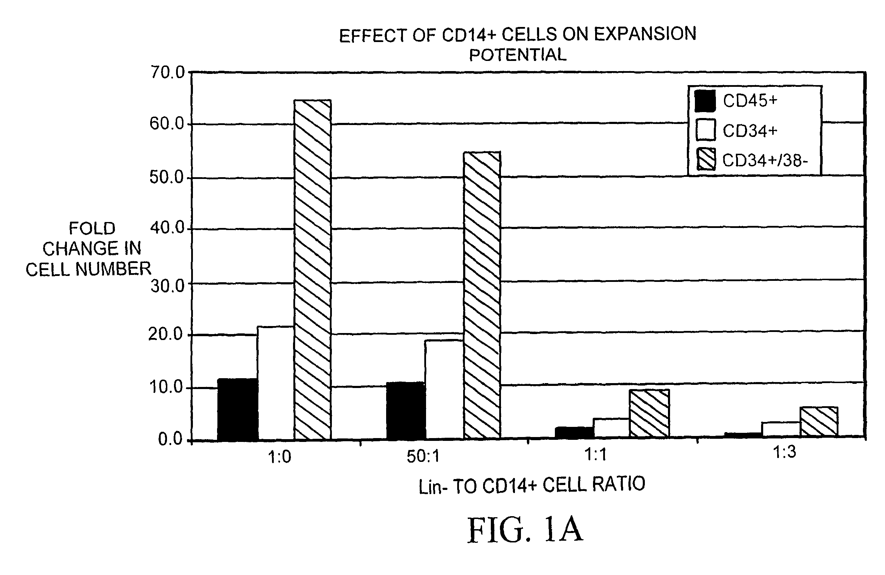

Cell products may be produced by providing a primary culture or initial cell suspension, and performing one or more selection steps (e.g., selection after cell division or proliferation has begun) using one or more antibody cocktails that remove a predetermined sub-population from the primary culture. The effect of various antibody cocktails on the expansion potential of a particular primary culture is shown in Fig. 1. In the experiment illustrated in Fig. 1, selection was performed prior to cell expansion. However, as will be discussed below in detail, selection with the antibody cocktails can be performed at any time and can be performed repeatedly before, during and after cell division.

Cell products may also be produced by (a) culturing a first population of cells that contains multiple cell types, including a first sub-population of cells, under conditions that cause the expansion of the first sub-population of cells, and (b) during or following expansion, removing from the first population a second sub-population of cells that are generated by the first sub-population, the infusable cell suspension including cells of the second sub-population. In some cases, the infusable cell suspension will be substantially free of cells of the first population, e.g., if such cells are not suitable for infusion. In some cases, for example in the case in which the first sub-population includes stem cells, the removing step is performed during expansion, and may be performed substantially continuously.

The composition of the antibody cocktail may be changed as time passes during cell expansion. For example, surface molecules expressed at different time points on different cells can be identified, and selection in or out can be based on this. Reselection with different antibody cocktails can be performed substantially continuously, or on an intermittent basis.

The proportions of different cells in the cell product can be adjusted, e.g., by adjusting antibody concentrations so that only a given proportion of non-target cells are selected out, and/or by changing the number and/or identity of the antibody or antibodies in the cocktail.

The antibody or antibodies in the cocktail can be selected to increase the fidelity of the product since some cell surface markers are shared by different cell types. For instance, CD45 positive cells bear at least several CD45 epitopes that represent unique subpopulations of leukocytes. As a result, a CD45 pan leukocyte marker can be used to select in or out all leukocytes, whereas CD45RA and CD45RO can be used to select in or out specific leukocyte subpopulations.

Proportions of cell types can also be varied by recombining the primary culture with sub-populations and/or progeny of sub-populations, during or after expansion.

Repeated or continuous selection of one or more sub-populations from the primary culture may be performed, the criteria for the selection being varied over time. For example, a particular sub-population of cells may be removed from the primary culture during initial selection steps, to enhance expansion of the primary culture, but not removed from the primary culture during later selection steps, to enhance the properties of the final cell product.

### Producing Cell Products

Some examples of suitable techniques for producing cell products that are suitable for infusion into a patient are discussed in detail below.

During cell culture, a first selection step may include selecting out a first sub-population that expresses CD14. This may be advantageous, as CD14 appears to inhibit the expansion of some cell populations, e.g., CD45+ cells, CD34+ cells and CD34+/38- cells, as shown in Fig. 1A.

From the first sub-population, a narrower, second sub-population that co-expresses CD14 and CD38 may be selected. This second sub-population is returned to the primary culture, after further expansion has occurred, or is added to the finished cell product. This may be desirable for several reasons.

First, the first sub-population may be undesirable in the culture, while the second sub-population is desirable. For example, the first sub-population may have a negative impact on culture kinetics, while the second sub-population may have an advantageous impact on culture kinetics and/or may favorably affect the potency of the suspension. Since the two sub-populations share a common selection marker, in order to separate the second sub-population from the first sub-population, so that the second sub-population may be returned to the culture, it is necessary to perform the two sequential selection steps described above. In this manner, the second sub-population can be isolated and returned to the culture without returning the undesirable first sub-population. If desired, e.g., if the first sub-population is detrimental to culture kinetics but advantageous to potency, the first sub-population can be returned at the conclusion of the expansion process.

If desired, the first and/or second sub-populations may be cultured separately prior to returning the second sub-population to the primary culture or adding it to the final cell product.

A sub-population can be removed from the primary culture and then returned after further expansion of the primary culture. This is done where the sub-population has an undesirable effect on expansion of the primary culture, but is useful in the final product. For example, CD14+ cells tend to inhibit expansion of a population of CD34+/CD38- cells, but may provide short-term support during the engraftment phase after delivery of the final product to a patient. The sub-population may be returned to the primary culture without further expansion of the sub-population, if desired.

The sub-population can also be cultured in a separate culture and then later returned to the primary culture. For example, again using CD34+/CD38- as the primary culture, a sub-population of CD7 lymphopoietic precursor cells can be removed and cultured under culture conditions that favor lymphopoietic cells. The expanded CD7 sub-population is then returned to the primary culture, e.g., to help reconstitution of lymphopoiesis of the immune system of the patient and thereby increase the kinetics of immune reconstitution.

If desired, in the above example CD3 cells can be separated out of the expanded CD7 sub-population before it is returned to the primary culture, to remove more differentiated lymphopoietic T-cells that may cause graft versus host disease (GVHD) in a patient.

During methods involving expansion of a sub-population, cells of the same type as those in the sub-population can be added to the sub-population culture to fuel expansion of the sub-population. These cells can be obtained by repeated selection from the primary culture as the primary culture continues to expand.

In some cases, rather than returning the sub-population to the primary culture, the sub-population can be added to a starting material from which the primary culture was obtained. For instance, the primary culture such as CD34+/CD38- can be obtained from cord blood and expanded, and subsequently added back to the cord blood to form a finished product. This would allow cord blood to be delivered to a large patient in need of a large number of CD34+/CD38- cells as well as other inherent constituents of cord blood.

It may also be desirable to remove cells of a sub-population from a primary culture, and add types other cells not of the primary culture to form a final product. For example, CD34+/CD38- cells and dendritic cells may be isolated from cord blood. The dendritic cells, which are difficult to expand, would be stored, while the CD34+/CD38- cells are cultured in a primary culture under conditions that will produce dendritic cells. The dendritic cells are then separated from the primary culture as a sub-population, and added to the dendritic cells that were isolated originally, to form a final product consisting of dendritic cells.

Dead cells may be removed from the primary culture and/or a sub-population separated from the primary culture and discarded. The resulting purified primary culture and/or sub-population can then be used as separate cell products or one combined cell product. For example, dead cells can be removed from a primary culture of CD34+/CD38- cells or a sub-population derived from this primary culture, and the purified culture or sub-population can be used as a final product.

Removal of dead cells from the primary culture has several advantages. Dead cells could impact the safety of the product, e.g., by causing undesirable reactions by the patient's immune system, or the potency of the other cells, and components released by dead cells could inhibit expansion or cause differentiation or apoptosis of the target cells. This is important because the resulting product will have a higher value because of higher purity and potency (both clinical and perceived improvement in potency and safety). In some preferred cell products, at least 85% of the cells present in the product are viable cells, more preferably at least 95% of the cells are viable.

### Selection Techniques

Preferred methods include selecting a sub-population of cells from cells in the primary culture, concurrently with proliferation, intermittently during proliferation or following proliferation. Cell proliferation and cell selection can be carried out using an almost infinite variety of different techniques and settings, of which only a few are described below by way of example. Many other techniques will be readily perceived by those skilled in the art, for example selection by flow cytometry, and selection by using chemical agents to kill unwanted cells. Selection may be performed using selection elements against cell surface markers. Positive or negative selection may be used, e.g., as described in U.S. Patent No. 5,925,567, the disclosure of which is incorporated herein by reference.

The preferred selection methods used in the invention can broadly be classed as positive selection (providing a selection element having an affinity for target cells) and negative selection (providing a selection element having an affinity for non-target cells). These two selection techniques, used alone or in combination, allow cells to be removed from the primary culture whenever desired, and also allow cells to be reselected from subpopulations to produce additional, narrower subpopulations.

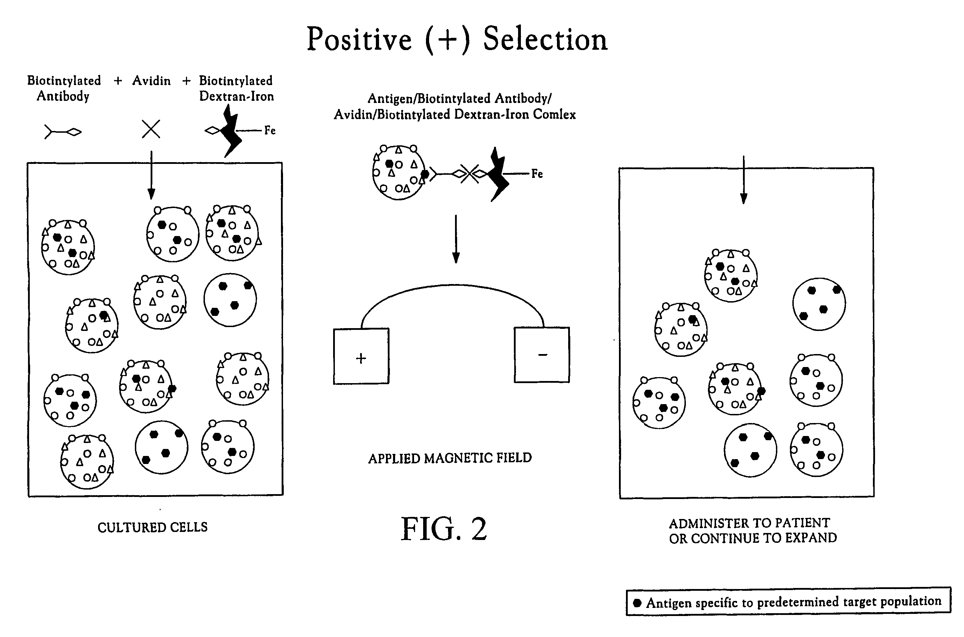

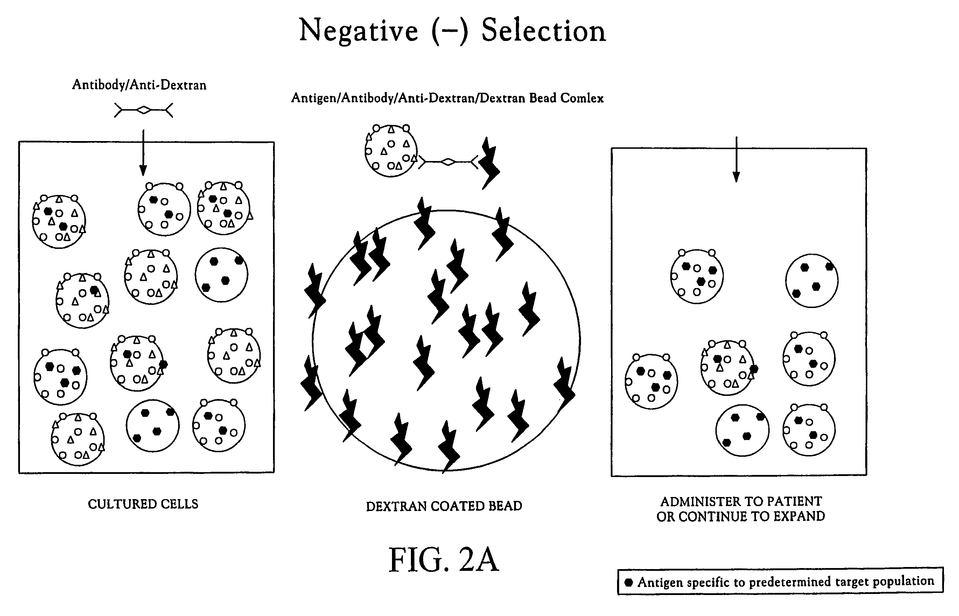

An example of a positive selection technique is illustrated diagrammatically in FIG. 2. Briefly, one or more anti-dextran conjugated antibodies specific for the predetermined target population is introduced into the culture. After a specified incubation time, a magnetic dextran iron particle colloid is introduced into the cell suspension. A Cell/Antigen/Antibody/Anti-dextran/Dextran/Iron Complex fomis. This complex is then passed through a magnetic field. Positively selected cells remain in the magnetic field while cells which do not have the iron conjugated complex are removed. After capture and rinsing the magnetic field is removed and the positively selected predetermined target population is returned to the nutrient medium.

An example of a negative selection technique is illustrated diagrammatically in FIG. 2A. Briefly, one or more anti-dextran conjugated antibodies specific for a predetermined population which is not of the predetermined target population is introduced into the culture. After a specified incubation time, a magnetic dextran iron particle colloid is introduced into the cell suspension. A Cell/Antigen/Antibody/Anti- Complex forms. This complex is then passed through a magnetic field, removing cells not of the predetermined target population from the nutrient medium. The predetermined target population is collected downstream and returned to the nutrient medium.

Clearly, many other techniques can be utilized for both positive and negative selection, as long as the desired affinity is provided by the selection element.

The selection element can include other components in addition to the antibody molecules that are used to perform the selection (the "selection molecules"), e.g., a solid support to which the selection molecule is bound. The solid support can be formed of a material that will aid in performing the selection or in maintaining the selection molecules in a desired position or introducing and removing them from the system. For example, as described above with reference to FIG. 2, the selection molecule can be bound to iron or other magnetic particles to allow the selected cells to be easily removed from the system by application of a magnetic field and then collected by removal of the magnetic field. Alternatively, the selection molecules can be bound onto the wall of a vessel containing the nutrient medium, or of a chamber through which the nutrient medium flows during the method. Glass or other inert, impermeable beads can also be used as a solid support. If beads or other particles are used, they can be provided in a packed configuration, through which the nutrient medium flows, or can be introduced into the system in a loose form, suspension, or in any desired type of array. As will be readily understood, a wide variety of other solid supports can be used.

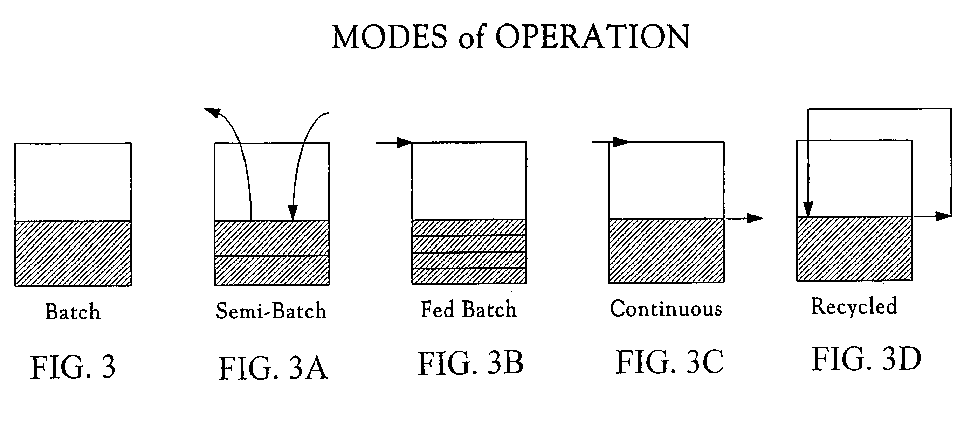

As shown in FIGS. 3-3D, the selection element can be used in a variety of modes of operation in which nutrient media is supplied to and removed from the system in different manners. These modes of operation range from a selective batch culture (FIG. 3), in which nutrient media is supplied at the beginning of cell proliferation and is neither added to nor removed, to continuous flow or recycled flow cultures (FIGS. 3C and 3D, respectively) in which either fresh or recycled nutrient media flows through the system substantially continuously. These alternative modes will be discussed in detail below.

In a selective batch culture (FIG. 3), a nutrient medium is introduced into a vessel, and a starting sample of cells is also introduced into the vessel. During cell proliferation, nutrient medium may or may not be exchanged. However, selected cells are physically selected, i.e., separated from other cells in the nutrient medium by binding to a selection element, either continuously, intermittently or following cell proliferation. These selected cells may be cells of a target population, if positive selection is used, or unwanted cells, if negative selection is used. Dual (positive and negative) selection can be accomplished by providing positive selection molecules on the surface of the vessel, beads, baffles, impellers, etc. while removing unwanted cells by negative selection. Alternatively, cells may be positively or negatively selected outside of the culture vessel and then returned.

The selective semi-batch (3A) and selective fed batch (3B) modes of operation are similar to the selective batch mode with regard to positive and negative selection. The significant difference between these three modes is in the treatment of the nutrient medium. While in the batch mode the volume of the medium remains constant and the medium is not refreshed (it may be supplemented), the semi-batch mode allows for a partial refreshment of spent medium with new medium and the fed batch mode allows for an incremental increase in the medium volume over time.

Cell growth and selection can also be performed in a continuous (FIG. 3C) or recycling (FIG. 3D) mode of operation. In these two modes, the system includes a chamber having an inlet and an outlet, and nutrient media is caused to flow through the chamber from the inlet to the outlet. In continuous mode, new nutrient media flows through the chamber from a source or reservoir, while in recycling mode the same nutrient media is cycled through the chamber repeatedly. If desired, a system can be configured to be run alternatively in either continuous or recycling mode. Any desired selection element can be used in these modes of operation.

### Example 1

Using a 9 antibody (CD2, CD3, CD14, CD16, CD19, CD24, CD56, CD66b, and GlyA) negative selection cocktail (Stemcell Tech) as a normalized control we tested the relationship between the initial antibody combination and the Total Cell and CD34+/CD38- cell output at day 7 post inoculation of lineage depleted Umbilical Cord Blood. Cultures contained Flt-3, SCF, and Tpo at 100 ng/ml each.

Referring to Fig. 1, the selection cocktails represented deviated from the 9 antibody control cocktail (3^{rd} set of bars) in several ways. From left to right, cocktails 1-14 were no GLYA or CD24 with the other 7 antibodies (Abs) of the control cocktail plus CD38 (N=2), no CD19 with the other 8 Abs (N=4), all 9 Abs (control; N=10), no CD16 with the other 8 Abs (N=4), no CD56 with the other 8 Abs (N=4), no CD66b with the other 8 Abs (N=4), no CD14 with the other 8 Abs (N=4), no CD2 with the other 8 Abs (N=4), no GlyA with the other 8 Abs (N=4), no CD24 with the other 8 Abs (N=9), no CD3 with the other 8 Abs (N=9), no GlyA or CD24 (N=4) with the other 7 Abs, no GlyA or CD3 with the other 7 Abs (N=4), and no GlyA or CD3 or CD24 with the other 6 Abs (N=8), respectively.

This combinatorial approach reveals the differential generation, in both the quantity and identity, of the process output. The selective expansion of distinct populations using various antibody cocktail selection strategies is critical given the impact of distinct populations on the production of target populations such as CD34+/CD38- cells. One example of this potential negative impact on the relative production of CD34+/CD38- cells can be ascribed to the presence of CD14+ cells in the configuration, no CD14 with the other 8 Abs (cocktail #7). This cocktail amplified CD34+/CD38- at 81.6% of the 9 antibody control (cocktail #3). Another example is the independent observation that the lack of CD38+ cells, in the presence of both GlyA+ and CD24+ cells, in the CD38 depleted culture (cocktail # 1) resulted in an amplification in CD34+/CD38- cells that was 66.9% of the 9 antibody control. This last observation is in contrast to observations regarding cocktails #9, #10, #12, and #14 that each lacked GlyA or CD24 or both. In these instances the amplification of CD34+/CD38- was increased by 113.1, 124.9, 107.2, and 126.4 respectively, relative to the 9 antibody control

### Example 2

This study was initiated based on the empirical finding that CD34+/CD38-cells expanded with less amplitude, 84.6% (N=4) of the control (N=10), if the Lin-cocktail (CD2/CD3/CD14/CD16/CD19/CD24/CD56/CD66b/GlyA-control) used to generate the inocula did not contain the antibody CD14. With this result we proceeded to test, in a dose dependent manner, the effect of CD14+ cells on the expansion of highly selected CD34+/CD38- populations. Umbilical Cord Blood was obtained and separated by magnetic means whereby CD14+ cells were first isolated by positive magnetic selection (Dynal) the cell suspension was then treated to isolate the target population using the 9 antibody control cocktail described above (StemCell Tech), except for the exclusion of the CD 14 antibody. Approximately equal fractions containing a combination of CD45+ (5.00x10⁵), CD34+ (6.84x10⁴), CD34+/CD38/Lin- (2.19x10⁴) cells from the same specimen were seeded with increasing numbers of CD14+ cells. The Lin- to CD14+ ratios were 1:0, 50:1, 1:1, and 1:3, respectively. With the four supplemental CD14+ cell values the initial number of CD45+ cells in each culture was 5.00x10⁵, 5.10x10⁵, 1.00x10⁶, and 2.00x10⁶, respectively. On day 7 of the cultures, cells were sampled to determine the absolute number of cells in each of three phenotype compartments, namely CD45, CD34 and CD34+/CD38- cells, relative to their initial numbers.

The results of this testing are shown in Fig. 1A. Each of the three compartments was sensitive to the presence of supplemental CD14+ cells at as low as a one (1) CD14+ cell per initial fifty (50) Lin- cells (or, 0.5% supplemented). Furthermore, increased concentrations of CD14+ cells led to the near abrogation of growth in the CD34+ and CD34+/CD38- compartments, with the mature CD45+ compartment being more or less annihilated at the highest CD 14+ cell ratio.

This result demonstrates that specific populations, such as the CD14+ cells, can significantly impact the ability to effectively expand the target population, in this case CD45+ cells. Despite this inhibitory effect, CD14+ cells may improve the effect of the graft on a patient, and thus it may be desirable to recombine the CD14+ cells with the target cells after expansion of the target population is complete.

## Claims

1. A method for producing a cell suspension suitable for engraftment or infusion comprising the steps of:
a) culturing a first population of cells that contains multiple cell types, including a first sub-population of cells under conditions that cause expansion of said first sub-population of cells,
b) before, during, or following the culturing step a), removing from said first population of cells a second sub-population of cells, wherein said second sub-population of cells inhibits said expansion of said first sub-population of cells or grows less well under said conditions than the cells of said first sub-population of cells and
c) further expanding said first population of cells and combining cells of said second sub-population with cells obtained by expansion of said first sub-population of cells to form the cell suspension.

2. The method of claim 1, wherein said second sub-population of cells is capable of enhancing engraftment of said cell suspension.

3. The method of claim 1, further comprising any one of the following options:
i) in step (c), combining cells of said second sub-population after expansion of said first sub-population is completed;
ii) removing dead cells;
iii) expanding said second sub-population prior to step c);
iv) in step (b), removing said second sub-population by using selection elements recognizing surface markers on the cells of said second sub-population.

4. The method of claim 1 wherein said initial cell suspension is derived from bone marrow, cord blood, peripheral blood or a primary culture obtained from human mesodermal, ectodermal and endodermal tissues

5. The method of claim 4, wherein said tissue is pancreatic, hepatic, neural, nephrotic, dermal, muscle or cardiac.

6. The method of claim 1, further introducing a third sub-population of cells selected from engraftment-enhancing cells, expansion-enhancing cells, or potency-enhancing cells to said cell suspension wherein said third sub-population of cells is added prior to completion of the expansion of the first sub-population of cells.

7. The method of claim 1, wherein removal of the second sub-population of cells in step (b) is performed during said culturing step a

8. The method of claim 7, wherein said removal is performed substantially continuously.

9. A method of producing a cell suspension suitable for infusion comprising :
a) obtaining an initial suspension of cells containing multiple cell populations;
b) selecting a population of target cells from said initial cell suspension;
c) culturing said population of target cells under conditions that cause the expansion of said target cells and
d) combining at least a portion of the product of step (c) with the remainder of said initial cell suspension to form the cell suspension suitable for infusion.

10. The method of claim 9, wherein said initial cell suspension is selected from the group consisting of bone-marrow, cord blood and peripheral blood.

11. The method of claim 9 wherein said initial cell suspension contains cells obtained from human mesodermal, ectodermal and endodermal tissues.

12. The method of claim 9, wherein said initial cell suspension contains target cells selected from the group consisting of: CD34+/CD38-multipotent progenitors, CD34+/CD7+ common lymphoid progenitors, CD34+/CD33+ common myeloid progenitors and combination thereof.

13. The method of claim 9, further comprising during step (c) removing from the culture a sub-population of cells that have an inhibitory effect on expansion of the target cells or grow less well under the culture conditions than said target cell, optionally further comprising after further expansion of the target cell culture, combining cells of said sub-population with the cultured target cells or the initial cell suspension, or more preferably further comprising expanding said sub-population.

14. The method of claim 9, further comprising selecting from either said initial cell suspension or said population of target cells, a sub-population of cells other than said target cells and optionally further comprising during step (d) combining at a least a portion of the cells of the sub-population with the initial cell suspension.

15. The method of claim 11, wherein said tissues are selected from the group consisting of pancreatic, hepatic, neural, nephritic, dermal, muscle and cardiac

## Patentansprüche

1. Verfahren zur Herstellung einer für eine Transplantation oder eine Infusion geeigneten Zellsuspension, umfassend die folgenden Schritte:
a) Kultivieren einer ersten Zellpopulation, welche multiple Zellarten enthält, einschließlich einer ersten Zellsubpopulation unter Bedingungen, die ein Wachsen genannter ersten Zellsubpopulation verursachen;
b) vor, während oder nach dem Kultivierungsschritt a), Entfernen einer zweiten Zellsubpopulation von genannter ersten Zellsubpopulation, wobei genannte zweite Zellsubpopulation genanntes Wachsen von genannter ersten Zellsubpopulation inhibiert oder unter den genannten Bedingungen weniger gut wächst als die Zellen der genannten ersten Zellsubpopulation, und
c) weiteres Wachsen lassen von genannter ersten Zellsubpopulation und Vereinigen der Zellen von genannter zweiten Subpopulation mit Zellen, die durch Wachsen der genannten ersten Zellsubpopulation erhalten wurden, um eine Zellsuspension zu bilden.

2. Verfahren gemäß Anspruch 1, in welchem genannte zweite Zellsubpopulation die Transplantation von genannter Zellsuspension verbessern kann.

3. Verfahren nach Anspruch 1, weiterhin umfassend irgendeine der folgenden Möglichkeiten:
i) in Schritt (c), Vereinigen von Zellen aus genannter zweiten Subpopulation, nachdem das Wachsen der ersten Subpopulation abgeschlossen ist;
ii) Entfernen von toten Zellen;
iii) Wachsen lassen von genannter zweiten Subpopulation vor Schritt c);
iv) in Schritt (b), Entfernen von genannter zweiten Subpopulation unter Verwendung von Auswahlelementen, die Oberflächenmarker auf den Zellen von genannter zweiten Subpopulation erkennen.

4. Verfahren gemäß Anspruch 1, in welchem genannte anfängliche Zellsuspension aus Knochenmark, Nabelschnurblut, peripherem Blut oder einer primären Kultur gewonnen wurde, die aus humanen mesodermalen, ektodermalen und endodermalen Gewebe erhalten wurden.

5. Verfahren gemäß Anspruch 4, in welchem genanntes Gewebe ein Pankreas-, Leber-, Neural-, Dermal-, Muskel- oder kardiales Gewebe ist.

6. Verfahren gemäß Anspruch 1, weiterhin einführend eine dritte Zellsubpopulation, ausgewählt aus transplantionsfördernden Zellen, wachstumsfördernden Zellen oder potenzfördernden Zellen, zu genannter Zellsuspension, wobei genannte dritte Zellsubpopulation vor dem Abschluss des Wachsens der ersten Zellsubpopulation zugegeben wird.

7. Verfahren gemäß Anspruch 1, in welchem die Entfernung der zweiten Zellsubpopulation in Schritt (b) während des genannten Kultivierungsschritt a) ausgeführt wird.

8. Verfahren nach Anspruch 7, wobei genannte Entfernung im Wesentlichen kontinuierlich ausgeführt wird.

9. Verfahren zur Herstellung einer Zellsuspension, die für Infusionen geeignet ist; umfassend:
a) Erhalten einer anfänglichen Zellsuspension, die multiple Zellpopulationen enthält;
b) Auswählen einer Zielzellpopulation aus genannter anfänglichen Zellsuspension;
c) Kultivieren genannter Zielzellpopulation unter Bedingungen, welche das Wachsen von genannten Zielzellen verursacht; und
d) Vereinigen von mindestens einem Teil des Produktes aus Schritt (c) mit dem Rest der genannten anfänglichen Zellsuspension, um eine für die Infusion geeignete Zellsuspension zu bilden.

10. Verfahren gemäß Anspruch 9, in welchem genannte anfängliche Zellsuspension ausgewählt wird aus der Gruppe bestehend aus Knochenmark, Nabelschnurblut und peripherem Blut.

11. Verfahren gemäß Anspruch 9, in welchem genannte anfängliche Zellsuspension Zellen enthält, die aus humanen mesodermalen, ektodermalen und endodermalen Gewebe erhalten werden.

12. Verfahren gemäß Anspruch 9, in welchem genannte anfängliche Zellsuspension Zielzellen enthalt, die ausgewählt werden aus der Gruppe bestehend aus: multipotenten CD34+/CD38-Progenitorzellen, gemeinsamen lymphoiden CD34+/CD7+-Progenitorzellen, gemeinsamen myloiden CD34+/CD33+-Progenitorzellen und Kombinationen daraus.

13. Verfahren gemäß Anspruch 9, während Schritt (c) weiterhin umfassend das Entfernen einer Zellsubpopulation aus der Kultur, die eine inhibitorische Wirkung auf das Wachsen der Zielzellen ausübt oder unter den Kulturbedingungen weniger gut wächst als genannte Zielzellen, gegebenenfalls weiterhin umfassend nach weiterem Wachsen der Zielzellkultur, Vereinigen von genannter Subpopulation mit den kultivierten Zielzellen oder der anfänglichen Zellsuspension, oder mehr bevorzugt weiterhin umfassend das Wachsen genannter Subpopulation.

14. Verfahren gemäß Anspruch 9, weiterhin umfassend die Auswahl von entweder genannter anfänglicher Zellsuspension oder genannter Zielzellpopulation, einer anderen Zellsubpopulation als von genannten Zielzellen und gegebenenfalls weiterhin umfassend während Schritt (d) Vereinigen von mindestens einem Teil der Zellsubpopulation mit der anfänglichen Zellsuspension.

15. Verfahren gemäß Anspruch 11, in welchem genannte Gewebe ausgewählt werden aus der Gruppe bestehend aus Pankreas-, Leber-, Neural-, Nieren-, Dermal-, Muskel- oder kardiales Gewebe.

## Revendications

1. Un procédé pour la production d'une suspension de cellules adapté à la prise de greffe ou l'infusion comprenant les étapes suivantes :
a) la mise en culture d'une première population de cellules contenant plusieurs types de cellules, comportant une première sous-population de cellules à des conditions provoquant l'expansion de ladite première sous-population de cellules,
b) avant, pendant ou après l'étape de mise en culture a) enlèvement d'une seconde sous-population de cellules de ladite première population de cellules , où ladite seconde sous-population de cellules empêche l'expansion de ladite première sous-population de cellules ou croit moins bien à de telles conditions que les cellules de la première sous-population de cellules, et
c) l'expansion supplémentaire de ladite première population de cellules et combinaison de cellules de ladite seconde sous-population avec des cellules obtenues par expansion de ladite première sous-population de cellules pour former la suspension de cellules.

2. Le procédé selon la revendication 1, où ladite seconde sous-population de cellules est capable d'augmenter la prise de greffe de ladite suspension de cellules.

3. Le procédé selon la revendication 1, comprenant en outre n'importe laquelle des options suivantes :
i) à l'étape (c) combinaison de cellules de ladite seconde sous-population dès que l'expansion de ladite première sous-population est terminée ;
ii) enlèvement des cellules mortes ;
iii) expansion de ladite seconde sous-population avant l'étape c) ;
iv) à l'étape b) enlèvement de ladite seconde sous-population en utilisant des éléments de sélection reconnaissant des générateurs de surface sur les cellules de ladite seconde population.

4. Le procédé selon la revendication 1, où ladite suspension initiale de cellules est dérivée de la moelle osseuse, du sang de cordon ombilical, du sang périphérique ou d'une culture primaire obtenue des tissus mésodermiques, ectodermiques et endodermiques humains.

5. Le procédé selon la revendication 4, où ledit tissu est pancréatique, hépatique, neural, néphrétique, dermique, musculaire ou cardiaque.

6. Le procédé selon la revendication 1, introduisant en outre une troisième sous-population de cellules sélectionnée à partir de cellules augmentant la prise de greffe, des cellules augmentant l'expansion, ou des cellules augmentant la dilution pour ladite suspension de cellules où ladite troisième sous-population de cellules est ajoutée avant la fin de l'expansion de la première sous-population de cellules.

7. Le procédé selon la revendication 1, où l'enlèvement de la seconde sous-population de cellules à l'étape (b) est effectué pendant ladite étape a) de mise en culture.

8. Le procédé selon la revendication 7, où ledit enlèvement est effectué essentiellement de façon continue.

9. Un procédé pour la production d'une suspension de cellules adapté à l'infusion comprenant les étapes suivantes :
a) obtention d'une suspension initiale de cellules contenant plusieurs populations de cellules ;
b) sélection d'une population de cellules cible à partir d'une suspension initiale de cellules ;
c) mise en culture de ladite population de cellules cibles à des conditions provoquant l'expansion des desdites cellules cibles et
d) combinaison d'au moins une partie du produit de l'étape (c) avec le reste de ladite suspension initiale de cellules pour former la suspension de cellules adaptée à l'infusion.

10. Le procédé selon la revendication 9, où ladite suspension initiale de cellules est sélectionnée à partir du groupe consistant de moelle osseuse, du sang de cordon ombilical et du sang périphérique.

11. Le procédé selon la revendication 9, où ladite suspension initiale de cellules contient des cellules obtenus à partir de tissus mésodermiques, ectodermiques et endodermiques humains.

12. Le procédé selon la revendication 9, où ladite suspension initiale de cellules contient des cellules cibles sélectionnés à partir le groupe consistant de : progéniteurs multipotents CD 34 + / CD 38 - , des progéniteurs lymphoïdes communs CD 34 + / CD 7 +, des progéniteurs myéloïdes communs CD 34 + / CD 33 + et leurs combinaisons.

13. Le procédé selon la revendication 9, comprenant en outre pendant l'étape (c) l'enlèvement de la culture une sous-population de cellules ayant un effet d'inhibition sur l'expansion des cellules cible ou croissant moins bien à de telles conditions que ladite cellule cible, comprenant de façon optionnelle, en outre, ensuite une expansion supplémentaire de la culture de cellules cible, une combinaison de cellules de ladite sous-population avec les cellules cible mises en culture ou la suspension initiale de cellules, ou comprenant plus préférée une expansion de ladite sous-population.

14. Le procédé selon la revendication 9, comprenant, en outre, une sélection à partir de ladite suspension initiale de cellules ou de ladite population de cellules cible, une sous-population de cellules différent desdites cellules cible, ou comprenant en outre de façon optionnelle pendant l'étape (d) une combinaison d'au moins une partie de cellules de ladite sous-population avec la suspension initiale de cellules.

15. Le procédé selon la revendication 11, où lesdits tissus sont sélectionnés à partir d'un groupe consistant de tissus pancréatiques, hépatiques, neuraux, néphrétiques, dermiques, musculaires ou cardiaques.
